# DEMANDE DE BREVET EUROPEEN

(11) **EP 2 025 304 A1**
(43) Date de publication de la demande: **18.02.2009**
(21) Numéro de dépôt: 08162283.9
(22) Date de dépôt: 13.08.2008
(51) Int. Cl.: A61F 2/00

(54) **Implant de soutien de l'urètre d'un homme et ensemble chirurgical de traitement de l'incontinence urinaire chez un homme comprenant un tel implant**

(30) Priorité: 17.08.2007 FR 0705884
(71) Demandeur: CL Medical, 69110 Sainte Foy Les Lyon (FR)
(72) Inventeur: Goria, Vincent, Jean-Claude, 69005 LYON (FR)
(74) Mandataire: Myon, Gérard Jean-Pierre

(57) **Abrégé**

Cet implant (2) comporte deux bandelettes souples allongées (20, 30) distinctes, sensiblement inextensibles suivant leur direction longitudinale et comprenant, d'une part, des parties médianes respectives (20C, 30C), qui sont disposées parallèlement l'une à côté de l'autre de manière adjacente le long du bord latéral d'une des deux bandelettes tourné vers l'autre bandelette, et au niveau desquelles les bandelettes, ainsi juxtaposées bord contre bord, sont liées mécaniquement l'une à l'autre, et, d'autre part, des parties intermédiaires respectives (20B, 30B, 20D, 30D) qui s'étendent entre la partie médiane et chaque extrémité longitudinale (20A, 30A, 20E, 30E) de chaque bandelette, et au niveau desquelles les bandelettes sont disjointes l'une de l'autre. Cet implant garantit ainsi un soutien obstructif fiable de l'urètre, les deux bandelettes pouvant être mises en tension indépendamment l'une de l'autre tandis que leurs parties médianes agissent sur l'urètre sur les largeurs cumulées des bandelettes.

## Description

La présente invention concerne un implant de soutien de l'urètre d'un homme pour traiter l'incontinence urinaire. Elle concerne également un ensemble chirurgical et une méthode de traitement de l'incontinence urinaire chez un homme, avec un tel implant.

L'incontinence urinaire chez l'homme après prostatectomie est une affection invalidante qui altère la qualité de la vie même pour une incontinence modérée. Le nombre de patients incontinents a augmenté ces dernières années avec l'avènement des possibilités de dépistage précoce du cancer de la prostate et la réalisation d'un nombre plus élevé de prostatectomies totales. Même si les techniques opératoires récentes permettent de mieux préserver le sphincter, des fuites d'urine peuvent survenir dans des pourcentages de cas non négligeables : ces fuites d'urine sont liées à la technique chirurgicale qui fait sectionner la partie basse de la prostate juste au voisinage du sphincter, mais peuvent également être favorisées par des éléments constitutifs liés au malade, comme une longueur d'urètre courte, un sphincter faible ou fatigable, etc.

Pour les patients souffrant d'une incontinence urinaire malgré une rééduction de leur sphincter, le traitement chirurgical couramment employé ces dernières années consiste à implanter un sphincter artificiel. Cette intervention limite significativement l'incontinence mais souffre d'inconvénients majeurs : le patient doit manipuler une pompe à chaque miction, le matériel implanté est onéreux et le taux de réintervention est élevé.

Une nouvelle technique chirurgicale de traitement est donc apparue ces toutes dernières années, à savoir la pose d'un implant de soutien sous-urétral, se présentant généralement à la façon d'un filet sous forme d'une plaque. Ce genre de filet nécessite d'être fermement amarré aux structures anatomiques du patient, pour garantir un soutien efficace de l'urètre et corriger ainsi l'incontinence. La plaque est ainsi soit suspendue par des fils remontant de chaque côté de la vessie par voie sus- ou rétro-pubienne, avec fixations terminales dans la paroi abdominale du patient, soit directement accrochée par des vis dans les branches osseuses ischiopubiennes du patient. Dans le premier cas, les risques de perforation vésicale sont réels et, dans le second cas, l'intervention chirurgicale, plus complexe, expose le patient à un risque d'ostéite. Dans les deux cas, la présence de la plaque implantée peut entraîner un inconfort périnéal transitoire postopératoire, voire des douleurs périnéales permanentes. De plus, même si à court terme, le taux de succès du traitement est élevé, il tend à baisser significativement au cours du temps.

Dans le même temps, des matériels et des méthodes de traitement de l'incontinence chez la femme ont été développés avec succès ces dernières années. En particulier, on connaît, par EP-A-1 342 450 au nom de la présente Demanderesse, une bandelette de soutien sous-urétrale, pouvant être implantée, entre autres, par une voie dite « transobturatrice », c'est-à-dire par passage des parties d'extrémité libre de la bandelette à travers respectivement les trous obturateurs de l'os iliaque d'une patiente, créant ainsi un effet de suspension pour l'urètre au niveau de la partie longitudinale médiane de la bandelette. Les intérêts de cette voie d'implantation transobturatrice sont réels, puisqu'elle ne fait courir aucun risque de perforation vésicale et ne nécessite aucune fixation osseuse, seules les extrémités libres de la bandelette étant amarrées dans l'abdomen de la patiente.

Le but de la présente invention est de proposer un implant de soutien de l'urètre d'un homme, qui soit efficace pour traiter l'incontinence urinaire, facile à manipuler lors de son implantation, et qui puisse être mis en place par une voix transobturatrice.

A cet effet, l'invention a pour objet un implant de soutien de l'urètre d'un homme pour traiter l'incontinence urinaire, caractérisé en ce qu'il comporte deux bandelettes souples allongées distinctes, sensiblement inextensibles suivant leur direction longitudinale et comprenant, d'une part, des parties médianes respectives, qui sont disposées parallèlement l'une à côté de l'autre de manière adjacente le long du bord latéral d'une des deux bandelettes tourné vers l'autre bandelette, et au niveau desquelles les bandelettes, ainsi juxtaposées bord contre bord, sont liées mécaniquement l'une à l'autre, et, d'autre part, des parties intermédiaires respectives qui s'étendent entre la partie médiane et chaque extrémité longitudinale de chaque bandelette, et au niveau desquelles les bandelettes sont disjointes l'une de l'autre.

L'implant conforme à l'invention, qui correspond en quelque sorte à une double bandelette, peut être implanté jusqu'à proximité de tissus biologiques sous-urétraux, en particulier à proximité du corps spongieux entourant l'urètre et des corps caverneux disposés de part et d'autre du corps spongieux. Sa forme spécifique à double bandelette permet, à la fois, de soutenir fermement et efficacement l'urètre et d'implanter l'implant par voix transobturatrice. Bien que la voie d'implantation transobturatrice mise en oeuvre avec l'implant selon l'invention puisse, de prime abord, être rapprochée de la voie transobturatrice mise en oeuvre dans le traitement de l'incontinence urinaire chez la femme, il convient de garder à l'esprit que les contraintes d'implantation chez l'homme sont radicalement opposées à celles de chez la femme : chez la femme, des bandelettes de soutien sous-urétrales sont implantées en restant souples au niveau du plancher pelvien, l'anatomie du bas ventre de la femme ne nécessitant et ne supportant aucune sollicitation trop ferme. Au contraire, chez l'homme, le corps spongieux entourant l'urètre présente une certaine fermeté, de sorte qu'un soutien efficace de l'urètre en vue de traiter l'incontinence nécessite un appui net et sous tension de l'implant contre ce corps spongieux, ou plus généralement des tissus anatomiques sous-urétraux, ce qui dissuade a priori l'homme du métier de chercher à transposer chez l'homme la technique transobturatrice mise au point chez la femme. Dans ces conditions, une des idées à la base de l'invention consiste à prévoir que, dans la zone médiane de l'implant, sa largeur, c'est-à-dire sa dimension prise suivant une direction sensiblement perpendiculaire à la direction longitudinale des deux bandelettes appartenant à l'implant, correspond à la somme des largeurs individuelles des bandelettes, en raison de la juxtaposition, bord contre bord, des parties médianes des bandelettes. Cette largeur doublée de la zone médiane de l'implant, par rapport au reste de l'implant, permet de réaliser un soutien urétral sur une étendue doublée par rapport à celle obtenue si l'on utilisait individuellement qu'une seule des deux bandelettes. De la sorte, lorsque l'implant est implanté, les parties médianes des deux bandelettes soutiennent alors le corps spongieux du patient sur une étendue substantielle et, par là, de manière ferme, y compris en soutenant le bulbe terminal de ce corps spongieux. Dans le même temps, chaque partie intermédiaire de chacune des deux bandelettes peut facilement, grâce à sa forme allongée de faible largeur, être passée à travers l'un des trous obturateurs de l'os iliaque du patient.

En outre, la structure spécifique de l'implant conforme à l'invention permet de le mettre en tension de manière différenciée pour chacune des deux bandelettes. Lors de l'implantation de l'implant, la traction sur les deux parties intermédiaires de chaque bandelette créée, dans le fond du U formé par la partie médiane de la bandelette correspondante autour des tissus sous-urétraux, un effet de soutien transversal à l'urètre, en vue de comprimer l'urètre pour l'obstruer partiellement et ainsi traiter l'incontinence. Cette mise en tension de chaque bandelette est liée à la propriété d'inextensibilité de la bandelette suivant sa direction longitudinale. On comprend que, lors de l'implantation, le chirurgien peut appliquer des tractions d'intensités différentes à chaque bandelette, pour générer un effet de soutien plus marqué au niveau de la partie médiane d'une des bandelettes par rapport à la partie médiane de l'autre bandelette. Cette disposition trouve par exemple un intérêt tout particulier chez un patient ayant subit une prostatectomie : dans ce cas, le chirurgien peut mettre la bandelette postérieure, c'est-à-dire celle des deux bandelettes la plus proche de la vessie du patient, sous une tension plus forte que la bandelette antérieure. De manière plus générale, grâce à l'implant conforme à l'invention, le chirurgien peut ajuster la mise en tension de l'implant de manière différenciée et indépendante pour les deux bandelettes de l'implant et ainsi commander de manière fine l'effet de soutien de l'urètre.

Une disposition dimensionnelle avantageuse est spécifiée à la revendication dépendante 8.

De la sorte, la zone médiane de l'implant présente une longueur suffisante pour obtenir l'effet de soutien urétral recherché, tout en étant facile à mettre en place lors de l'implantation de l'implant. Les structures anatomiques du patient peuvent alors être dégagées profondément, sans compliquer les gestes du chirurgien, car de part et d'autre de cette zone médiane de l'implant, les parties intermédiaires des deux bandelettes présentent une faible largeur, notamment par rapport à des implants de type plaque.

Un mode de réalisation pratique, à la fois facile à fabriquer et à implanter, est défini à la revendication 9.

Grâce à l'aménagement avantageux défini à la revendication 2, les deux parties intermédiaires des bandelettes, situées d'un même côté des parties médianes, sont conjointement manipulées lors de l'implantation de l'implant, en particulier pour mettre en place ces deux parties intermédiaires à travers l'un des trous obturateurs de l'os iliaque du patient en un seul passage.

D'autres caractéristiques avantageuses de l'implant conforme à l'invention, prises isolément ou suivant toutes les combinaisons techniquement possibles, sont spécifiées aux revendications dépendantes 3 à 7.

L'invention a également pour objet un ensemble chirurgical de traitement de l'incontinence urinaire chez un homme tel que défini à la revendication 10.

On divulgue également ici une méthode chirurgicale de traitement de l'incontinence chez un homme, au moyen d'un implant de soutien de l'urètre d'un patient, cet implant comportant deux bandelettes souples allongées distinctes, sensiblement inextensibles suivant leur direction longitudinale et comprenant, d'une part, des parties médianes respectives, qui sont disposées parallèlement l'une à côté de l'autre de manière adjacente le long du bord latéral d'une des deux bandelettes tourné vers l'autre bandelette, et au niveau desquelles les bandelettes, ainsi juxtaposées bord contre bord, sont liées mécaniquement l'une à l'autre, et, d'autre part, des parties intermédiaires respectives, qui s'étendent entre la partie médiane et chaque extrémité longitudinale de chaque bandelette, et au niveau desquelles les bandelettes sont disjointes l'une de l'autre,
ladite méthode comprenant des étapes peropératoires consistant à :
i) inciser verticalement la peau et la graisse sous-cutanée de la région périnéale du patient, entre ses bourses et son anus, jusqu'au bulbe de son corps spongieux urétral, tout en préservant ce corps spongieux ;
ii) dans l'incision périnéale réalisée lors de l'étape i), dégager, de chaque côté latéral du corps spongieux urétral, les deux corps caverneux du patient ;
iii) inciser sagittalement l'aponévrose périnéale entre chacun des deux corps caverneux et le corps spongieux urétral ;
iv) mettre en place l'implant dans le corps du patient, de manière que les parties médianes des bandelettes s'étendent au-dessous et transversalement au corps spongieux urétral tandis que, de chaque côté de ces parties médianes, les parties intermédiaires correspondantes des deux bandelettes s'étendent de l'incision correspondante de l'aponévrose périnéale réalisée lors de l'étape iii) jusqu'à la racine de la cuisse correspondante du patient, en passant par le trou obturateur correspondant de l'os iliaque du patient, les extrémités longitudinales des bandelettes s'étendant à l'extérieur du patient depuis les racines des cuisses ;
v) tirer sur les parties intermédiaires des bandelettes de manière à mettre les parties médianes en appui tendu contre le corps spongieux urétral, les intensités de traction appliquées respectivement aux deux bandelettes étant ajustables de manière indépendante l'une de l'autre ; et
vi) sectionner les parties des bandelettes qui s'étendent à l'extérieur de la racine des cuisses et fermer l'incision périnéale réalisée à l'étape i).

L'invention sera mieux comprise à la lecture de la description qui va suivre, donnée uniquement à titre d'exemple et fait en se référant aux dessins sur lesquels :
- la figure 1 est une vue schématique en perspective d'un ensemble chirurgical selon l'invention ;
- la figure 2 est une vue schématique en élévation du détail encadré II à la figure 1 ;
- la figure 3 est une vue schématique en perspective du détail encadré III à la figure 1 ;
- la figure 4 est une section schématique sagittale du bas-ventre d'un homme ;
- la figure 5 est une vue en élévation prise selon la flèche V de la figure 4, illustrant la mise en oeuvre de l'ensemble de traitement de la figure 1 ; et
- la figure 6 est une section schématique selon la ligne VI-VI de la figure 5.

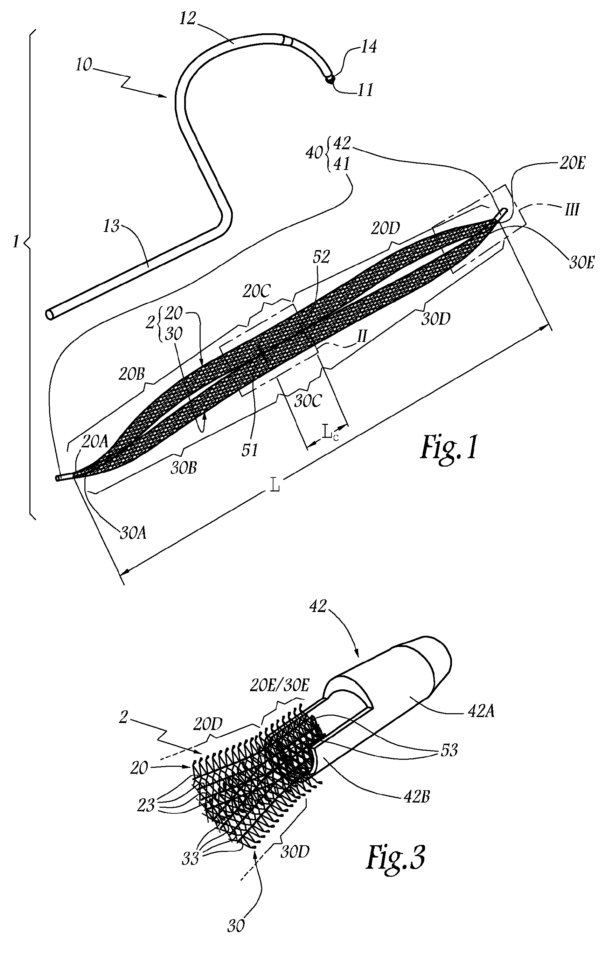

Sur la figure 1 est représenté un ensemble 1 de traitement de l'incontinence urinaire chez un homme. Cet ensemble 1 comprend un implant 2 destiné à soutenir l'urètre d'un patient et une aiguille incurvée 10.

L'aiguille 10 est en acier chirurgical et comprend, successivement suivant sa longueur, une extrémité 11 pointue, une partie courante 12 incurvée et une partie de base 13 plus particulièrement affectée à la préhension de l'aiguille. Une poignée facultative, non représentée, peut ainsi être rapportée de manière amovible sur la partie de base 13. L'aiguille utilisée peut présenter des formes de réalisation diverses, notamment en ce qui concerne sa partie incurvée 12 : dans l'exemple représenté à la figure 1, cette partie 12 s'étend globalement dans un seul plan, avec un profil partiellement circulaire dans ce plan, mais d'autres géométries sont aussi envisageables, telles qu'une forme partiellement hélicoïdale.

L'implant 2 comporte essentiellement deux bandelettes distinctes 20 et 30 et des moyens 40 de solidarisation de l'implant à l'aiguille 10, décrits en détail plus loin. Dans l'exemple considéré ici, les bandelettes 20 et 30 sont identiques l'une à l'autre.

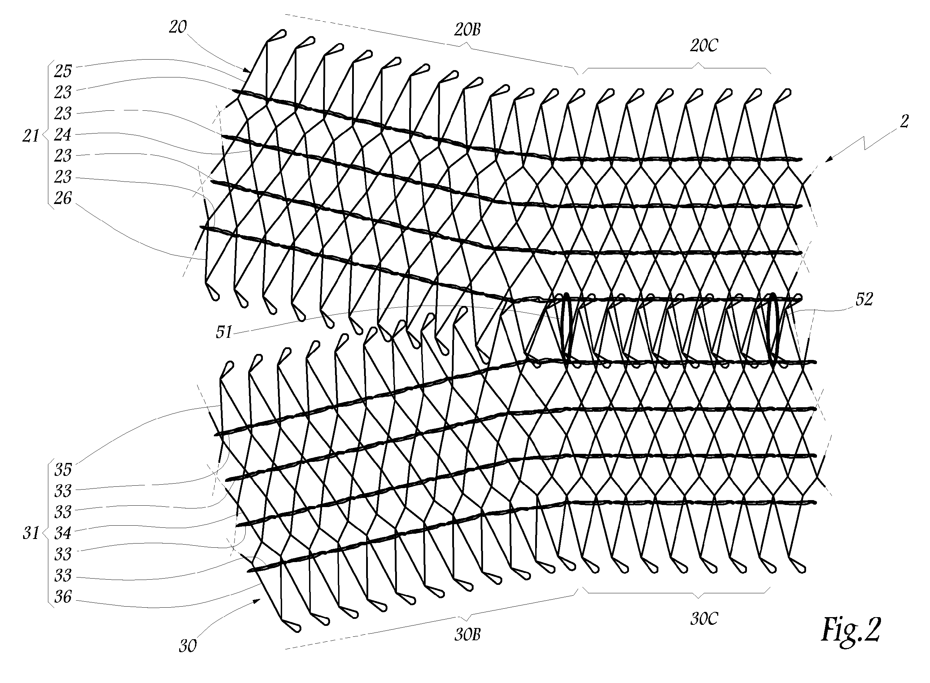

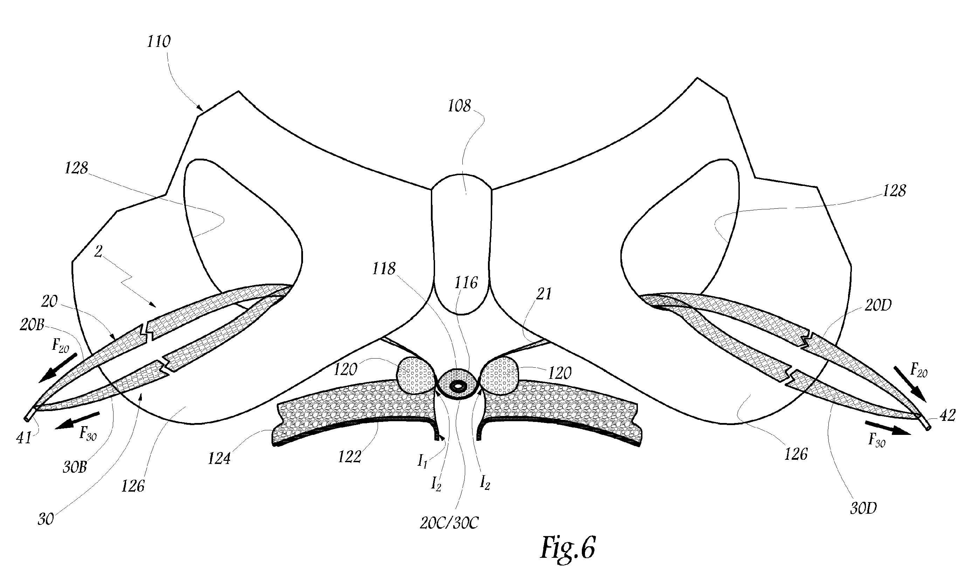

Chaque bandelette 20, 30 est constituée, sur toute sa longueur, d'un tricot 21, 31 de fils biocompatibles, de préférence de monofilaments de polypropylène. Sur les figures 1 et 6, la structure des tricots n'est pas visible, au profit d'une représentation très schématique à des fins d'illustration, tandis que la représentation des tricots sur les figures 2, 3 et 5 permet d'apprécier les détails de cette structure. Chaque tricot 21, 31 comprend, d'une part, quatre chaînettes longitudinales tricotées 23, 33, parallèles entre elles et formant l'armature du tricot et, d'autre part, un treillis intermédiaire maillé 24, 34, reliant transversalement les chaînettes. Cette structure du tricot permet à ce dernier d'être à la fois souple et non extensible, pour permettre sa mise en place dans des tissus biologiques mous du patient, en adaptant sa forme à celle de ces tissus, tout en conférant au tricot une élasticité sensiblement nulle suivant la direction longitudinale de la bandelette, pour permettre d'implanter cette dernière sous une forte tension longitudinale.

Chaque tricot 21, 31 inclut également deux franges latérales opposées 25 et 26, 35 et 36, bordant sur toute leur longueur les deux côtés latéraux de la bandelette 20 et 30 à la façon de lisières. Chaque frange 25, 26, 35, 36 est constituée de fils du tricot 21, 31, qui s'étendent vers l'extérieur de la bandelette et de manière transversale à la chaînette de bordure 23, 33 correspondante. Ces franges sont ainsi à même de s'accrocher aux tissus biologiques lorsque l'implant 2 est implanté dans le corps du patient. Chacun des fils constituant ces franges présentent avantageusement la forme d'une boucle fermée, pour limiter l'irritation des tissus biologiques lors de la mise en place de l'implant.

En ce qui concerne la liaison des bandelettes 20 et 30 l'une à l'autre lorsque l'implant 2 est prêt à être manipulé par un chirurgien pour être implanté, chaque bandelette définit, suivant sa longueur, cinq parties successives 20A à 20E, 30A à 30E, comme précisé ci-après.

Les bandelettes 20 et 30 sont liées mécaniquement l'une à l'autre, à la fois, au niveau de leurs parties médianes 20C et 30C, au niveau d'une de leurs extrémités longitudinales, référencée 20A et 30A, et au niveau de leur extrémité longitudinale opposée 20E et 30E.

Plus précisément, au niveau des parties médianes 20C et 30C des bandelettes 20 et 30, deux des chaînettes de bordure 23 et 33, appartenant respectivement aux bandelettes 20 et 30 et tournées l'une vers l'autre, sont reliées l'une à l'autre par couture, par l'intermédiaire de deux lignes de points de couture 51 et 52, espacées l'une de l'autre suivant la direction longitudinale des bandelettes, comme visible à la figure 1 et comme représenté plus en détail à la figure 2. Chaque ligne 51, 52 s'étend en longueur suivant une direction sensiblement perpendiculaire aux deux chaînettes précitées, de telle sorte que les parties médianes 20C et 30C des bandelettes s'étendent l'une le long de l'autre de manière adjacente, avec leur chaînette de bordure respective 23 et 33 s'étendant en regard l'une de l'autre de manière parallèle. Entre ces deux chaînettes de bordure en regard, la partie correspondante de la frange 26 est entremêlée avec la partie correspondante de la frange 35, par enchevêtrement des fils constituant ces franges, sans toutefois que ces fils ne soient noués les uns aux autres.

Les extrémités 20A et 30A des bandelettes 20 et 30 sont liées l'une à l'autre par un élément 41, tandis que les extrémités 20E et 30E sont liées l'une à l'autre par un autre élément 42, comme visible à la figure 1 et comme représenté plus en détail pour l'élément 42 à la figure 3. Dans l'exemple de réalisation considéré ici, les éléments 41 et 42 sont identiques : chacun de ces éléments se présente sous la forme d'un manchon creux rigide, dont la partie d'extrémité opposée aux bandelettes 20 et 30 (référencée 42A pour l'élément 42 à la figure 3) est adaptée pour s'encliqueter autour de l'extrémité pointue 11 de l'aiguille 10, en particulier dans une rainure circonférentielle 14 ménagée dans le corps de l'aiguille au niveau de cette extrémité. La partie d'extrémité de chaque élément 41, 42, opposée à la partie d'extrémité (référencée 42B pour l'élément 42 à la figure 3), se présente sous la forme d'une portion de tube, à l'intérieur de laquelle les extrémités de bandelette 20A et 30A, 20E et 30E sont cousues, en particulier par au moins une ligne 53 de points de couture qui assemble les chaînettes 23 et 33 à la paroi de l'élément 41, 42.

Ainsi, chaque élément 41, 42 permet de solidariser conjointement les deux bandelettes 20 et 30 à l'aiguille 10, ces deux éléments appartenant donc ainsi aux moyens précités 40. Pour disposer d'une réalisation compacte, les extrémités de bandelette 20A et 30A, 20E et 30E sont globalement superposées l'une à l'autre à l'intérieur de la partie d'extrémité 41B, 42B de leur élément correspondant 41, 42, ce qui limite également le nombre de points de couture de la ou des lignes 53 nécessaire à la liaison mécanique des tricots 21 et 31 à cet élément.

Entre son extrémité 20A, 30A et sa partie médiane 20C, 30C, chaque bandelette 20, 30 définit une partie intermédiaire allongée 20B, 30B. Comme les bandelettes 20 et 30 sont liées mécaniquement l'une de l'autre, à la fois au niveau de leur partie médiane 20C, 30C et de leur extrémité 20A, 30A, les parties intermédiaires de bandelette 20B et 30B s'étendent globalement en regard l'une de l'autre, tout en étant disjointes l'une de l'autre, dans le sens où elles ne sont pas reliées directement l'une à l'autre, mais sont libres d'être écartées l'une de l'autre, comme représenté sur les figures 1 et 2.

Entre son extrémité 20E, 30E et sa partie médiane 20C, 30C, chaque bandelette 20, 30 définit une partie intermédiaire allongée 20D, 30D. Ces parties intermédiaires 20D et 30D sont fonctionnellement analogues aux parties intermédiaires 20B et 30B, de sorte que, au niveau de ces parties 20D et 30D, les bandelettes sont disjointes.

Pour obtenir l'implant 2, un procédé de fabrication particulièrement avantageux consiste à tricoter simultanément les bandelettes 20 et 30 de manière adjacente l'une le long de l'autre, avec la frange 26 entremêlée à la frange 35 sur toute la longueur des bandelettes. Puis on coud les bandelettes l'une à l'autre par l'intermédiaire des lignes 51 et 52. On sépare ensuite les deux bandelettes l'une de l'autre, entre leur extrémité 20A, 30A et la ligne 51 d'une part et entre leur extrémité 20E, 30E et la ligne 52 d'autre part. Pour se faire, on démêle les fils constituant les lisières 26 et 35 en écartant transversalement les bandelettes l'une de l'autre, en commençant au niveau des extrémités 20A et 30A, 20E et 30E puis en progressant vers la ligne 51, respectivement 52. On comprend que les lignes 51 et 52 forment des zones d'arrêt de la séparation des deux bandelettes, de sorte que les parties médianes de bandelette 20C et 30C demeurent parallèles et adjacentes l'une le long de l'autre et que les parties des franges 36 et 25 s'étendant au niveau de ces parties médianes demeurent entremêlées l'une avec l'autre. Les extrémités de bandelette 20A et 30A sont ensuite cousues à l'élément 41, tandis que les extrémités de bandelettes 20E et 30E sont cousues à l'élément 42.

On comprend de cet exemple de procédé de fabrication de l'implant 2, que les lignes de couture 51 et 52 définissent respectivement, le long des bandelettes 20 et 30, les extrémités longitudinales des parties médianes 20C et 30C, dans le sens où, entre ces deux lignes, les chaînettes 23 et 33 des tricots 21 et 31 sont maintenues sensiblement parallèles les unes aux autres et les franges 36 et 25 sont maintenus entremêlées, tandis que, du côté de la ligne 51, opposé à la ligne 52, et du côté de la ligne 52, opposé à la ligne 51, les tricots 21 et 31 sont disjoints, avec notamment les franges 36 et 25 désengagées l'une de l'autre, pour former ainsi les parties intermédiaires de bandelette 20B, 30B, 20D et 30D. L'effet de maintien des parties médianes de bandelette 20C et 30C par les lignes de couture 51 et 52 est en pratique lié au fait que la distance séparant ces deux lignes, c'est-à-dire la longueur L_{C} des parties médianes 20C et 30C, est plus petite que la longueur de chacune des parties intermédiaires 20B, 30B, 20D, 30D, en constituant notamment moins de 20% de la longueur totale L des bandelettes 20 et 30. Suivant un exemple de réalisation avantageux, la longueur L_{C} est inférieure à 8 cm et de préférence égale à 3 cm ± 1 cm, pour des raisons anatomiques qui apparaîtront plus loin.

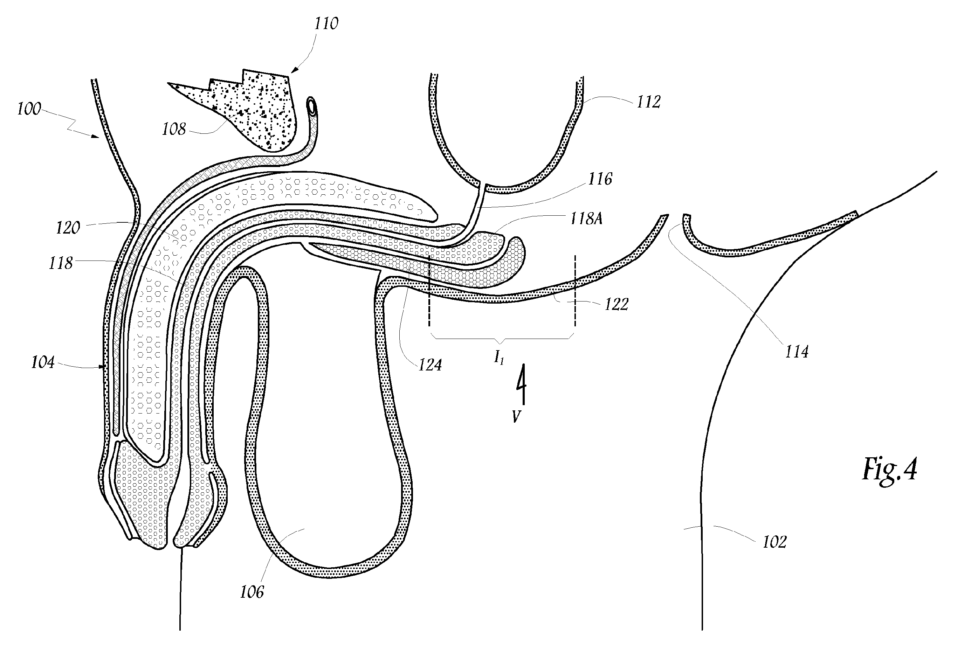

Sur la figure 4, on voit de manière schématique le bas-ventre 100 d'un homme, ainsi que, en arrière-plan, le contour de la partie haute d'une de ses cuisses 102. Dans le plan sagittal correspondant à cette figure, apparaissent successivement, de l'avant vers l'arrière du patient, sa verge 104, le contour d'une de ses bourses 106, le pubis 108 de son os iliaque 110, sa vessie 112 et son anus 114. L'écoulement d'urine depuis la vessie 112 jusqu'à l'extérieur de la verge 104 est réalisé par l'urètre 116 du patient. La majeure partie longitudinale de l'urètre est entourée par le corps spongieux 118 de la verge 104, de part et d'autre duquel s'étendent en longueur les deux corps caverneux 120 de la verge. Sur la figure 4, un seul des corps caverneux est représenté, étant entendu que les deux corps caverneux sont situés de part et d'autre et de manière sensiblement symétrique au plan sagittal du patient, tandis que l'urètre 116 et le corps spongieux 118 s'étendent de manière globalement centrée sur ce plan.

On va maintenant décrire une méthode chirurgicale de traitement de l'incontinence urinaire chez le patient dont le bas-ventre 100 est représenté à la figure 4, au moyen de l'ensemble 1 des figures 1 à 3.

Juste avant l'intervention chirurgicale proprement dite, le patient, sous anesthésie, est sondé par une sonde urétro-vessicale et installé dans une position gynécologique.

Le chirurgien incise verticalement la région périnéale du patient, entre, à l'arrière, l'anus 114 et, à l'avant, les bourses 106, comme indiqué par la référence I₁ sur les figures 4 à 6. La limite avant de l'incision I₁ est donnée par le repérage, avec le doigt, du bord inférieur du pubis 108, tandis que le milieu de l'incision est repéré par le coude que forme l'urètre 116 en direction de la vessie 112, ce coude étant mis en relief par la sonde urétro-vessicale. L'incision I₁ traverse vers le haut à la fois la peau 122 et la graisse sous-cutanée 124 de la région périnéale, et est poursuivie jusqu'à atteindre le bulbe terminale 118A du corps spongieux 118, qui est toutefois préservé. De chaque côté latéral de ce bulbe, on voit alors apparaître les reliefs arrondis blanchâtres de l'albuginée des corps caverneux 120. Par palpation, de chaque côté du bulbe 118A, le chirurgien met alors en évidence le dièdre entre chaque corps caverneux 120 et le corps spongieux 118.

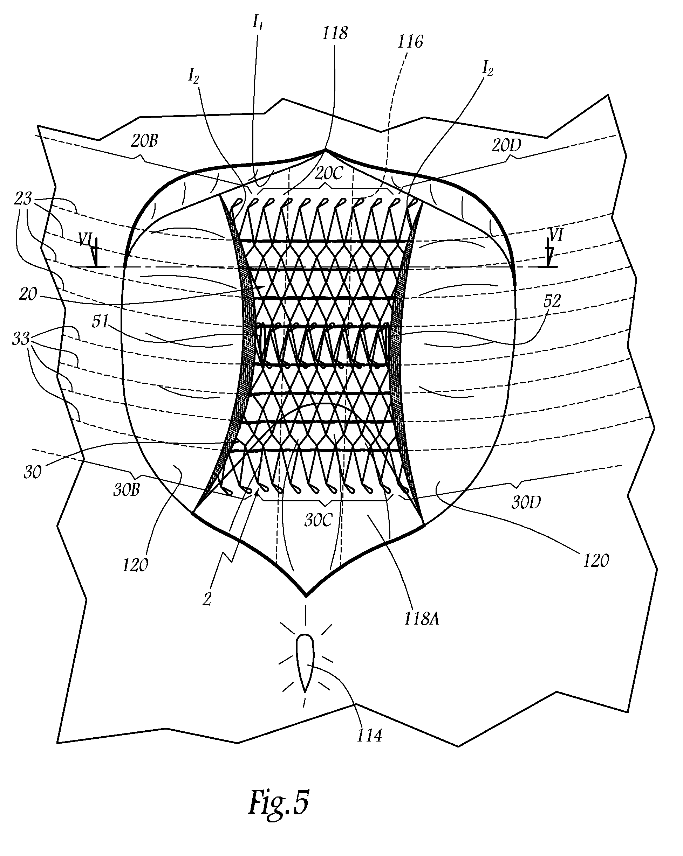

Au fond de chaque dièdre ainsi mis en évidence, l'aponévrose périnéale est incisée de manière sensiblement sagittale, par exemple à l'aide d'un bistouri électrique, sur une profondeur d'environ 1 cm, si possible sans entrer dans le corps caverneux correspondant 120, comme indiqué par les références I₂ sur les figures 5 et 6. Au doigt, le chirurgien palpe et libère doucement les tissus incisés de l'aponévrose, en contournant par dessus chaque corps caverneux 120, pour aller au contact de la branche ischio-pubienne correspondante 126 de l'os iliaque 110 (figure 6).

Le chirurgien se saisit ensuite de l'aiguille 10 et l'introduit dans le corps du patient, à partir de la racine d'une première de ses cuisses 102. Le point d'entrée de l'extrémité pointue 11 de l'aiguille au niveau de la racine de la cuisse est avantageusement situé à la fois à environ 4 cm de l'incision I₁ et à environ 4 cm du bord inférieur du relief du muscle grand adducteur de la cuisse. Grâce à sa partie courante incurvée 12, l'aiguille 10 progresse dans les tissus du patient tout en restant du même côté latéral du patient que sa première cuisse 102, jusqu'à atteindre le trou obturateur correspondant 128 de l'os iliaque, à travers lequel l'extrémité pointue 11 progresse jusqu'à rejoindre l'incision I₂ correspondante. Pour ce faire, le chirurgien introduit un doigt dans l'incision I₂, de manière à percevoir l'extrémité pointue 11 en approche et guider ainsi sa sortie dans cette incision.

Une fois que l'extrémité pointue 11 de l'aiguille a franchi l'incision I₂, l'implant 2 est fixé sur l'aiguille grâce à l'élément de solidarisation 41 encliqueté dans la rainure 14, puis l'aiguille est ressortie en la tirant par sa partie de base 13, de sorte que l'extrémité 11 suit la trajectoire inverse l'ayant amenée jusqu'à la région périnéale. Les bandelettes 20 et 30 sont ainsi mises en place dans le corps du patient entre une première des deux incisions I₂ et la racine de la première cuisse 102 du patient, en passant par un premier des deux trous obturateurs 128 de l'os iliaque 110. L'aiguille 10 est ainsi tractée vers l'extérieur jusqu'à ce que les extrémités de bandelette 20A et 30A s'étendent à l'extérieur du patient depuis la racine de sa cuisse. Le chirurgien dégage alors les bandelettes de l'aiguille, notamment en déconnectant l'élément 41.

Avec la même aiguille 10 ou une aiguille analogue, le chirurgien réalise des gestes symétriques pour le second côté latéral du patient. L'aiguille est introduite depuis la racine de la seconde cuisse 102, jusqu'à ce que son extrémité pointue 11 rejoigne et s'étende à l'extérieur de la seconde incision I₂ de l'aponévrose entre le second corps caverneux 120 et le corps spongieux 118, en passant par le second trou obturateur 128. Puis les extrémités de bandelette 20E et 30E sont solidarisées à l'extrémité pointue 11, au moyen à l'élément 42. Par traction et retrait de l'aiguille, les bandelettes 20 et 30 sont mises en place dans le corps du patient entre la seconde incision I₂ et la racine de la seconde cuisse 102, en passant par le second trou obturateur 128 et avec les extrémités de bandelette 20E et 30E s'étendant à l'extérieur du patient depuis la racine de sa cuisse. Les bandelettes 20 et 30 sont alors globalement dans la configuration d'implantation illustrée aux figures 5 et 6, c'est-à-dire avec leur partie médiane 20C et 30C s'étendant en longueur de manière globalement perpendiculaire au plan sagittal médian de l'urètre 116.

Le chirurgien met alors sous tension chaque bandelette 20, 30, c'est-à-dire, comme indiqué par les flèches F₂₀, F₃₀ à la figure 6, qu'il tire sur les parties intermédiaires 20B et 20D, 30B et 30D de chaque bandelette, en agissant en pratique sur les terminaisons de ces parties s'étendant à l'extérieur de la racine des cuisses du patient, de manière que la partie médiane 20C, 30C de la bandelette s'appuie de manière franche contre le corps spongieux 118, par le dessous de ce corps. Chaque partie 20C, 30C présente alors une forme de U, au fond duquel le corps spongieux 118 est soutenu. La tension exercée sur l'implant 2 est réglée de manière à obtenir un soutien obstructif de l'urètre 116, afin de traiter l'incontinence. Pour se faire, le chirurgien tend avantageusement les bandelettes 20 et 30 avec des intensités différentes, en particulier pour tenir compte de la morphologie et de la pathologie du patient. Ainsi, à titre d'exemple, dans le cas d'un patient ayant subit une prostatectomie, la bandelette implantée la plus proche de la vessie 112, c'est-à-dire la bandelette 20 dans l'exemple considéré aux figures 5 et 6, est mise en place avec une tension d'intensité plus forte que la bandelette 30, pour soutenir fermement, voire comprimer légèrement le bulbe 118A. A l'inverse, dans certains cas cliniques, une mise en tension plus intense de la bandelette antérieure peut être privilégiée, notamment pour limiter les risques de migration ultérieure de l'implant 2 vers l'arrière.

Dans tous les cas d'ajustement de la tension des bandelettes 20 et 30, on comprend que les parties médianes de bandelette 20C et 30C forment à elles seules la zone de soutien de l'urètre 116, ce qui explique qu'une valeur de l'ordre de 3 cm pour la longueur L_{C} est judicieuse. A ce propos, le fait que la liaison par couture au niveau de ces parties 20C et 30C soit réalisée exclusivement aux extrémités longitudinales de ces parties, par les lignes 51, 52, évite que des points de couture ne soient trop fermement pressés contre les tissus sous-urétraux et favorise un comportement homogène de chaque bandelette 20, 30 sur la longueur de sa partie médiane 20C, 30C. En outre, grâce à l'implant 2, on agit sur l'urètre 116 au niveau d'une largeur d'implant doublée par rapport à la largeur individuelle des bandelettes 20 et 30, tout en mettant à profit la largeur individuelle modérée des bandelettes pour leur passage par la voix transobturatrice décrite ci-dessus, en limitant ainsi autant que possible l'étendue transversale des tissus biologiques traversés par ces bandelettes.

Une fois que le chirurgien a réglé la tension de l'implant 2, ce dernier peut, si besoin, être ancré dans les tissus biologiques du patient pour éviter le relâchement de l'effet sur l'urètre 116. Cet ancrage n'est pas toujours nécessaire selon la morphologie et la pathologie du patient à traiter car les franges 25, 26, 35 et 36 au niveau des parties intermédiaires de bandelette 20B, 30B, 20D et 30D tendent à immobiliser les bandelettes dans les tissus biologiques traversées par ces bandelettes, par accrochage à ces tissus. Lorsqu'un ancrage additionnel est souhaité, le chirurgien utilise un fil de suture et accroche l'un et/ou l'autre des tricots 21 et 31 à des tissus du patient. Préférentiellement, ce sont les parties médianes 20C et 30C qui sont ainsi accrochées au corps spongieux 118, en des zones ponctuelles d'accrochage des chaînettes 23 et/ou 33 au corps 118. Avantageusement, ces zones d'accrochage sont au nombre de quatre et forment un motif en rectangle dont la médiatrice antéropostérieure est sensiblement située dans le plan sagittal médian de l'urètre 116. D'autres nombres et d'autres motifs pour ces zones d'accrochage sont envisageables.

L'intervention chirurgicale prend ensuite fin : les parties terminales des bandelettes 20 et 30, extériorisées au niveau des racines des cuisses 102, sont sectionnées et l'incision périnéale I₁ est refermée par plans, avec si besoin la fermeture préalable de chaque incision I₂.

En variante à la méthode chirurgicale décrite ci-dessus, l'étape de traction des bandelettes 20 et 30 pour ajuster leur tension et l'étape éventuelle d'accrochage par suture de ces bandelettes peuvent être inversées ou réalisées de manière concomitante.

Divers aménagements et variantes à l'ensemble 1 et à la méthode de traitement décrits ci-dessus sont sans doute envisageables. A titre d'exemples :
- pour l'implant 2 considéré sur les figures, les bandelettes 20 et 30 présentent une même largeur, sensiblement constante sur toute la longueur des bandelettes, ce qui constitue une forme de réalisation pratique à fabriquer et à manipuler lors de l'implantation ; toutefois, l'une des deux bandelettes peut, en variante non représentée, présenter une largeur supérieure à celle de l'autre bandelette, tout comme il n'est pas exclu que la largeur de l'une et/ou l'autre des bandelette varie selon sa longueur ; ainsi, un nombre supérieur ou inférieur à quatre est possible pour ce qui concerne les chaînettes 23 et/ou 33 des tricots 21 et 31 ;
- pour l'implant 2 décrit jusqu'ici, la liaison mécanique entre les bandelettes 20 et 30 repose sur les lignes de couture 51, 52 et 53, ce qui met avantageusement et efficacement à profit la structure tricotée de ces bandelettes ; il n'est toutefois pas exclu, en particulier si la propriété d'inextensibilité des bandelettes 20 et 30 repose sur une autre structure qu'un tricot, que les liens mécaniques au niveau des parties médianes de bandelette et de chaque extrémité de bandelette, soient réalisés par d'autres moyens, par exemple par un matériau adhésif, une gaine de rétreint, etc. ;
- diverses formes de réalisation sont envisageables pour ce qui concerne les moyens de solidarisation 40 ; le lecteur pourra se reporter au document EP-A-1 342 450 au nom de la Demanderesse pour connaître des détails et des variantes de réalisation de ces moyens, ainsi que les aménagements correspondants de l'aiguille 10 ; et/ou
- les aiguilles du type de l'aiguille 10 envisagée ci-dessus sont préférentiellement utilisées pour implanter l'implant 2 dans le corps d'un patient, étant entendu que d'autres ancillaires permettant la mise en place des bandelettes 20 et 30 peuvent être utilisés.

## Revendications

1. Implant (2) de soutien de l'urètre (116) d'un homme pour traiter l'incontinence urinaire, **caractérisé en ce qu'**il comporte deux bandelettes souples allongées (20, 30) distinctes, sensiblement inextensibles suivant leur direction longitudinale et comprenant, d'une part, des parties médianes respectives (20C, 30C), qui sont disposées parallèlement l'une à côté de l'autre de manière adjacente le long du bord latéral d'une des deux bandelettes tourné vers l'autre bandelette, et au niveau desquelles les bandelettes, ainsi juxtaposées bord contre bord, sont liées mécaniquement l'une à l'autre, et, d'autre part, des parties intermédiaires respectives (20B, 30B, 20D, 30D) qui s'étendent entre la partie médiane et chaque extrémité longitudinale (20A, 30A, 20E, 30E) de chaque bandelette, et au niveau desquelles les bandelettes sont disjointes l'une de l'autre.

2. Implant suivant la revendication 1, **caractérisé en ce que** les deux bandelettes (20, 30) sont également liées mécaniquement l'une à l'autre au niveau de leurs deux extrémités longitudinales (20A, 30A, 20E, 30E), qui sont chacune pourvues d'un élément (41, 42) de solidarisation conjointe des deux bandelettes à un ancillaire d'implantation de l'implant (2), en particulier à une aiguille d'implantation (10).

3. Implant suivant l'une des revendications 1 ou 2, **caractérisé en ce que** chaque bandelette (20, 30) est essentiellement constituée d'un tricot de fils (21, 31), comprenant des chaînettes d'armature longitudinales (23, 33) et un treillis intermédiaire (24, 34) reliant transversalement ces chaînettes.

4. Implant suivant les revendications 2 et 3 prises ensemble, **caractérisé en ce que,** au niveau de chaque extrémité longitudinale (20A, 30A, 20E, 30E) des bandelettes (20, 30), les tricots (21, 31) des deux bandelettes sont cousus à l'élément de solidarisation conjointe (41, 42).

5. Implant suivant l'une des revendications 3 ou 4, **caractérisé en ce qu'**au moins une des chaînettes (23, 33) appartenant à la partie médiane (20C, 30C) de l'une des bandelettes (20, 30) est reliée par couture à au moins l'une des chaînettes appartenant à la partie médiane de l'autre bandelette.

6. Implant suivant la revendication 5, **caractérisé en ce que** les chaînettes (23, 33) des parties médianes (20C, 30C) des deux bandelettes (20, 30) sont reliées par deux lignes de points de couture (51, 52), qui sont orientées transversalement aux chaînettes et qui définissent respectivement les extrémités longitudinales des parties médianes.

7. Implant suivant l'une quelconque des revendications 3 à 6, **caractérisé en ce que** chaque bandelette (20, 30) est bordée de deux franges latérales (25, 26, 35, 36) d'accrochage de tissus biologiques dans lesquels l'implant (2) est implanté, et **en ce que**, au niveau des parties médianes (20C, 30C) des deux bandelettes, les deux franges (26, 35) tournées l'une vers l'autre sont entremêlées.

8. Implant suivant l'une quelconque des revendications précédentes, **caractérisé en ce que** les parties médianes (20C, 30C) des bandelettes (20, 30) présentent une dimension longitudinale (L_{C}) inférieure à 8 cm, de préférence comprise entre 2 et 4 cm.

9. Implant suivant l'une quelconque des revendications précédentes, **caractérisé en ce que** la partie médiane (20C, 30C) et les parties intermédiaires (20B, 20D, 30B, 30D) de chaque bandelette (20, 30) présentent une même largeur, sensiblement constante suivant la longueur de la bandelette.

10. Ensemble chirurgical (1) de traitement de l'incontinence urinaire chez un homme, **caractérisé en ce qu'**il comporte :
- un implant (2) de soutien de l'urètre (116) d'un patient, conforme à l'une quelconque des revendications 2 à 9, et
- au moins un ancillaire d'implantation de l'implant, en particulier une aiguille d'implantation (10), adapté pour coopérer avec l'un et/ou l'autre des deux éléments de solidarisation conjointe (41, 42).
